# FASCICULE DE BREVET EUROPEEN

(11) **EP 0 708 629 B1**
(45) Date de publication et mention de la délivrance du brevet: **01.04.1998**
(21) Numéro de dépôt: 94922895.1
(22) Date de dépôt: 08.07.1994
(51) Int. Cl.: A61F 13/15

(54) **ARTICLE D'HYGIENE ABSORBANT JETABLE A DOUBLE BARRIERE D'ETANCHEITE ET PROCEDE DE FABRICATION**
ABSORBIERENDER HYGIENISCHER WEGWERFARTIKEL MIT ZWEI FLÜSSIGKEITSDICHTEN BARRIEREN SOWIE HERSTELLUNGSVERFAHREN DAFÜR
DISPOSABLE ABSORBENT SANITARY ARTICLE HAVING TWO LIQUID-TIGHT BARRIERS, AND METHOD FOR MANUFACTURING SAME

(30) Priorité: 12.07.1993 FR 9308566
(43) Date de publication de la demande: 01.05.1996
(73) Titulaire: SCA Mölnlycke, 59497 Linselles Cédex (FR)
(72) Inventeur: VANDEMOORTELE, Philippe, F-59200 Lille (FR)
(74) Mandataire: Casalonga, Axel
(86) Numéro de dépôt international: FR9400857
(87) Numéro de publication internationale: WO9502381

(56) Documents cités:
- EP-A- 0 518 044
- EP-A- 0 567 105
- WO-A-92/22271
- FR-A- 2 566 631
- FR-A- 2 695 314
- US-A- 4 846 825

## Description

La présente invention se rapporte à un article d'hygiène absorbant jetable, tel qu'une couche-culotte ou garniture pour incontinent, du type comprenant un coussin absorbant disposé entre une feuille extérieure de support imperméable et une feuille intérieure perméable.

Plus particulièrement la présente invention concerne un tel article d'hygiène absorbant jetable comprenant une double barrière d'étanchéité constituée par une première barrière d'étanchéité périphérique formant poche de confinement et disposée sensiblement sur toute la périphérie du coussin absorbant, complétée par une deuxième barrière d'étanchéité formée de deux revers longitudinaux, verticaux, situés de part et d'autre de la première barrière périphérique.

Des articles d'hygiène sont connus par exemple par la demande de brevet GB-A 2 161 059 (Kimberly-Clark). Suivant ce document deux volets d'étanchéité s'étendent, au-dessus du coussin absorbant, dirigés l'un vers l'autre de manière que leurs bords distaux élastifiés soient situés à proximité l'un de l'autre. Les volets latéraux d'étanchéité sont réalisés par un double pliage de la feuille intérieure perméable aux liquides recouvrant la feuille de support imperméable aux liquides ainsi que le coussin absorbant fixé sur cette feuille de support. A leurs deux extrémités correspondant aux deux bords de la couche-culotte, les volets sont fixés de manière à être toujours tournés vers l'intérieur, l'un vers l'autre, sans pouvoir se plier vers l'extérieur par exemple lors de la mise en place de l'article d'hygiène sur l'utilisateur. Ces volets latéraux améliorent l'étanchéité des articles d'hygiène dans le sens latéral notamment dans la zone d'entre-jambes. En revanche, des risques de fuites subsistent dans les deux zones de ceinture.

Il est par ailleurs connu par exemple par la demande de brevet GB-A 2 159 693 (Kimberly-Clark), de prévoir dans chaque zone de ceinture d'un tel article d'hygiène, une poche constituée par un volet s'étendant transversalement depuis un bord longitudinal de l'article d'hygiène en direction du bord longitudinal opposé, le bord le plus interne de ce volet pouvant éventuellement être élastifié. Ces poches améliorent l'étanchéité uniquement dans les zones de ceinture.

Il a déjà été proposé d'améliorer, sur un même article d'hygiène, à la fois l'étanchéité latérale, notamment dans la zone d'entre-jambes, et l'étanchéité dans les zones de ceinture. Ainsi suivant le brevet US-A-4 662 877 (Williams), la feuille intérieure d'un tel article d'hygiène est recouverte d'une feuille supplémentaire comportant au-dessus du coussin absorbant une découpe rectangulaire dont les deux bords longitudinaux sont élastifiés. Il est à noter que suivant ce document, la feuille supplémentaire est fixée à la feuille intérieure perméable suivant deux bords transversaux opposés correspondant aux bords transversaux de l'article d'hygiène et suivant deux bord longitudinaux opposés situés à l'extérieur des éléments élastiques d'entre-jambes de l'article d'hygiène, lesquels éléments élastiques sont eux-mêmes situés à l'extérieur des bords longitudinaux opposés du coussin absorbant. Un telle feuille supplémentaire comportant une ouverture ou découpe rectangulaire à bords longitudinaux élastifiés, entraîne une consommation importante de matière, en particulier du fait des chutes de matière résultant de la formation de la découpe rectangulaire ménagée dans la feuille supplémentaire. Or, dans le cas d'articles d'hygiène absorbants à jeter comme les couches-culottes il est essentiel de réduire le plus possible les quantités de matériau utilisé, et donc le prix de revient des articles.

La demande de brevet EP-A-0 219 326 (Procter & Gamble) décrit une couche en une seule pièce à jeter comprenant une feuille de support imperméable, un coussin absorbant et une feuille de couverture perméable recouvrant le coussin, cette feuille de couverture étant pourvue de deux volets latéraux longitudinaux formant barrières anti-fuites. Ces volets peuvent être constitués par des bandes rapportées fixées à la feuille de support le long de leurs bords longitudinaux proximaux et fixées à la feuille de couverture le long de leurs bords longitudinaux distaux à proximité des bords transversaux de la couche. Dans une autre réalisation, ces volets sont constitués par un double pliage de la feuille de couverture elle-même, le pli résultant du double pliage étant fixé le long de son bord longitudinal proximal à la feuille de couverture à l'extérieur du coussin et en partie le long de son bord longitudinal distal, à proximité des bords transversaux de la couche à la partie sous-jacente de la feuille de couverture. Ces volets latéraux améliorent l'étanchéité de la couche dans le sens latéral, notamment dans la zone d'entre-jambes. En revanche, des risques de fuites subsistent encore dans les zones de ceinture.

Pour remédier à ce défaut on a également proposé d'ajouter à la structure de la couche de la demande précédente deux poches de ceinture. En particulier la demande de brevet EP-A-0 376 022 (Procter & Gamble) décrit la réalisation de telles poches de ceinture qui sont constituées par deux bandes transversales rapportées. Une telle structure complique la fabrication des couches-culottes, notamment lorsque ces dernières sont fabriquées en continu dans le sens longitudinal. De plus cette structure accroît la consommation de matière nécessaire à la fabrication de la couche-culotte.

Le document US-A-4 846 825, representant le préambule des revendications 1, 9 et 16, décrit un article d'hygiène absorbant jetable, tel qu'une couche-culotte ou garniture pour incontinent, comprenant une feuille de support imperméable aux liquides, un coussin absorbant fixé sur la feuille de support, une feuille de couverture perméable aux liquides recouvrant le coussin absorbant, des éléments élastiques fixés à l'état tendu à la feuille de support à l'extérieur des bords longitudinaux du coussin, et deux volets latéraux fixés à la feuille de support symétriquement par rapport à un axe longitudinal médian de l'article. Ces volets sont uniformes, ne comportent pas de plis orientés verticalement et il n'existe pas de double barrière d'étanchéité.

Enfin, dans la demande de brevet FR-A 2 695 314 (Peaudouce) on propose une couche-culotte absorbante jetable comprenant une feuille extérieure de support imperméable, un coussin absorbant et une feuille de couverture intérieure perméable recouvrant le coussin absorbant qui comporte deux volets latéraux s'étendant au-dessus du coussin absorbant de sorte que leurs bords proximaux soient reliés à la feuille de couverture suivant des lignes longitudinales situées au-dessus des bords longitudinaux du coussin absorbant, lesdits volets latéraux étant reliés entre eux à leurs bords distaux depuis des bords transversaux de la couche-culotte au moins jusqu'aux bords transversaux correspondant du coussin absorbant, pour former ainsi une barrière d'étanchéité périphérique sur tout le pourtour du coussin. Il n'est pas prévu dans cette structure de revers ou barrières latérales d'étanchéité supplémentaires. D'autre part l'ouverture de la poche formée par les volets latéraux est de faible largeur, et il n'est pas possible de faire varier la largeur de cette ouverture dans la direction longitudinale, sauf à prévoir une découpe de largeur variable dans les volets, ce qui se traduit par un supplément de consommation de matière.

La présente invention a donc pour objet de fournir un article d'hygiène absorbant jetable, tel qu'une couche-culotte ou garniture pour incontinent comprenant une double barrière d'étanchéité, ne nécessitant qu'une consommation limitée de matière.

La présente invention a également pour but de fournir un tel article d'hygiène absorbant jetable comprenant une double barrière d'étanchéité constituée par une première barrière d'étanchéité périphérique sur toute la périphérie du coussin absorbant et formant poche de confinement complétée par une deuxième barrière d'étanchéité latérale formée de deux revers latéraux, verticaux, situés de part et d'autre de la première barrière d'étanchéité périphérique.

La présente invention a encore pour but un tel article d'hygiène absorbant comportant une barrière d'étanchéité périphérique formant poche de confinement dont la largeur varie dans la direction longitudinale.

La présente invention a aussi pour but un tel article d'hygiène absorbant dont les revers latéraux sont réalisés par des plis élastifiés formés à partir des volets eux-mêmes et ont une hauteur déterminée.

La présente invention a encore pour but un tel article d'hygiène absorbant dont la hauteur des plis formant les revers varie longitudinalement à partir d'une hauteur maximale.

La présente invention a enfin pour objet un procédé de fabrication d'un tel article d'hygiène absorbant qui soit simple et économique à mettre en oeuvre.

Selon la présente invention, l'article d'hygiène absorbant jetable, tel qu'une couche-culotte ou garniture pour incontinent, comprend une feuille de support imperméable aux liquides ayant des bords transversaux et des bords longitudinaux opposés, les bords longitudinaux comportant chacun une échancrure définissant une zone d'entre-jambes de largeur réduite et deux zones d'extrémité de largeur accrue. Un coussin absorbant, de forme généralement rectangulaire et de dimension inférieure à la feuille de support, est fixé sur ladite feuille de support. Une feuille de couverture intérieure, perméable aux liquides, de forme généralement rectangulaire est fixée au coussin absorbant. Cette feuille de couverture est de dimension suffisante pour recouvrir le coussin absorbant au moins dans la zone d'entre-jambes. Des premiers éléments élastiques longitudinaux sont fixés, à l'état tendu, à la feuille de support à l'extérieur des bords longitudinaux du coussin absorbant. Deux volets latéraux, en matériau souple, par exemple en non-tissé hydrophobe de forme générale rectangulaire et de longueur supérieure à la longueur du coussin absorbant, sont fixés à la feuille de support symétriquement par rapport à un axe longitudinal médian de l'article. Chacun des volets comprend une partie intermédiaire de longueur au moins égale à la zone d'entre-jambes et inférieure à la longueur du coussin absorbant réunissant deux parties d'extrémité. Les parties d'extrémité d'un volet sont réunies aux parties d'extrémités correspondantes de l'autre volet, le long de l'axe longitudinal médian. Les parties intermédiaires de chacun des volets comportent chacune un pli ou revers longitudinal, identique, de longueur au moins égale à celle de la zone d'entre-jambes et de hauteur (H) déterminée, orienté verticalement vers l'intérieur de l'article et situé au-dessus du coussin absorbant plus près du bord longitudinal du coussin absorbant dans la zone d'entre-jambes que de l'axe longitudinal médian. Des seconds éléments élastiques de longueur au moins égale à celle de la zone d'entre-jambes sont fixés, à l'état tendu, à l'intérieur de chacun desdits plis longitudinaux. Enfin, chacun des volets latéraux est fixé à la feuille de couverture par une ligne de liaison longitudinale située à l'extérieur par rapport audit pli longitudinal et au-dessus du coussin absorbant dans la zone d'entre-jambes.

Ainsi, lesdites parties intermédiaires forment conjointement une ouverture longitudinale de forme oblongue au-dessus du coussin absorbant qui constitue une première barrière d'étanchéité périphérique sur pratiquement le pourtour du coussin absorbant et comportent des revers latéraux orientés verticalement au-dessus du coussin absorbant constituant une deuxième barrière latérale d'étanchéité.

Dans une réalisation recommandée les parties d'extrémité des volets ont une largeur supérieure à la moitié de la largeur de la feuille de support de manière à présenter des zones en recouvrement le long de l'axe longitudinal médian, ces zones en recouvrement étant liées entre elles.

L'article d'hygiène absorbant selon l'invention comprend des troisièmes éléments élastiques longitudinaux fixés, à l'état tendu, le long de l'ouverture. Egalement, ces troisièmes éléments élastiques ont une longueur au moins égale à la longueur de la zone d'entre-jambes et sont disposés dans des goussets longitudinaux formés le long des bords longitudinaux de l'ouverture.

La hauteur déterminée (H) des plis longitudinaux de chacun des volets peut soit être constante sur toute la longueur des plis, soit diminuer longitudinalement régulièrement de part et d'autre d'un point de hauteur maximale jusqu'aux deux extrémités du pli. Ce point de hauteur maximale peut être situé au milieu du pli, c'est-à-dire qu'il divise longitudinalement le pli en deux parties égales ou être situé en tout endroit approprié suivant la longueur du pli, et dans ce cas il divise le pli longitudinalement en deux parties inégales, dont la hauteur diminue régulièrement vers les extrémités du pli. Comme indiqué, les plis de chacun des volets latéraux d'un même article sont identiques, c'est-à-dire qu'ils sont symétriques par rapport à un plan vertical passant par l'axe longitudinal médian de l'article.

Dans une réalisation particulièrement recommandée, les plis longitudinaux sont formés par des boucles fermées réalisées dans les parties intermédiaires des volets et sont situés plus près des bords longitudinaux du coussin absorbant dans la zone d'entre-jambes que de l'axe longitudinal médian de l'article.

Lorsque la hauteur (H) du pli de chacun des volets est constante sur pratiquement toute la longueur du pli, les parties intermédiaires de chacun des volets ont une largeur réduite, essentiellement constante, par rapport aux parties d'extrémité, sur pratiquement toute leur longueur. Les parties intermédiaires forment ainsi une ouverture de forme oblongue sur le coussin absorbant, de largeur pratiquement constante sur toute la longueur de la zone d'entre-jambes. Bien évidemment, en modifiant la hauteur des plis on modifie la largeur de l'ouverture, plus la hauteur (H) du pli est grande plus large est l'ouverture. Lorsque les plis ont une hauteur diminuant depuis un point de hauteur maximale, la largeur de l'ouverture diminuera avec la diminution de hauteur du pli à partir d'une largeur maximale d'ouverture correspondant aux points de hauteur maximale des plis. Les volets forment alors, une ouverture de forme ovale ou ovoïde, symétrique ou asymétrique par rapport à la largeur maximale de l'ouverture, selon que le point de hauteur maximale des plis divise ces plis longitudinalement en deux parties égales ou inégales. On peut aussi aisément réaliser une poche de confinement ou barrière périphérique dont la plus grande largeur se trouve dans la zone appropriée de l'article d'hygiène.

La présente invention concerne également un procédé de fabrication d'un volet destiné à être utilisé dans un article d'hygiène absorbant tel que décrit ci-dessus qui comporte les étapes consistant à :
- réaliser une bande en matériau souple, de préférence en non-tissé hydrophobe, de forme généralement rectangulaire ayant une largeur supérieure à la moitié de la largeur de l'article d'hygiène et de longueur analogue à celle de l'article d'hygiène;
- fixer longitudinalement, par rapport à un bord de la bande, à l'état tendu, de préférence à une distance inférieure à la moitié de la largeur de la bande, au moins un élément élastique sur une longueur au moins égale à la longueur de la zone d'entre-jambes de l'article;
- replier la bande sur elle-même, autour de l'élément élastique d'une hauteur déterminée, pour former une boucle ayant un sommet et des parois latérales longitudinales dont les bords longitudinaux inférieurs sont distants du sommet de ladite hauteur déterminée;
- amener en contact lesdits bords longitudinaux inférieurs de la boucle; et
- lier entre eux lesdits bords longitudinaux inférieurs suivant une ligne de liaison longitudinale pour former ainsi un volet ayant une partie intermédiaire, pourvue d'un pli formant revers vertical, de longueur au moins égale à la longueur de la zone d'entre-jambes reliant deux parties d'extrémité de plus grande largeur.

Les bords inférieurs longitudinaux de la boucle sont réunis par tous moyens appropriés, tels que collage, thermoscellage et soudure aux ultra-sons.

Bien évidemment, selon l'article final souhaité, on repliera la bande de façon à former une boucle de hauteur constante ou diminuant longitudinalement vers les extrémités.

Le procédé de fabrication recommandé du volet comprend en outre une étape consistant à fixer longitudinalement, à l'état tendu, un autre élément élastique sur une longueur au moins égale à la longueur de la zone d'entre-jambes le long d'un bord longitudinal de la bande.

On replie le bord longitudinal de la bande autour de cet autre élément élastique et on le scelle pour former un gousset contenant ledit autre élément élastique. La réalisation d'un tel gousset élastifié est classique et est décrite en particulier dans la demande de brevet FR-A-2695314.

La présente invention concerne encore un procédé de fixation de volets latéraux sur un article d'hygiène absorbant jetable comprenant une feuille de support imperméable aux liquides ayant des bords transversaux et des bords longitudinaux opposés, les bords longitudinaux comportant chacun une échancrure définissant une zone d'entre-jambes de largeur réduite et deux zones d'extrémité de largeur accrue, un coussin absorbant fixé sur la feuille de support et de dimension inférieure à celle-ci, une feuille de couverture, perméable aux liquides, de forme générale rectangulaire, fixée au coussin absorbant et de dimension suffisante pour recouvrir le coussin absorbant au moins dans la zone d'entre-jambes et des premiers éléments élastiques longitudinaux fixés, à l'état tendu, à la feuille de support, à l'extérieur des bords longitudinaux du coussin absorbant, consistant à :
- réaliser deux volets identiques comme décrit ci-dessus;
- disposer les volets ainsi réalisés symétriquement par rapport à un axe longitudinal médian au-dessus de la feuille de support, du coussin absorbant et de la feuille de couverture, de telle sorte que les boucles soient situées au-dessus du coussin absorbant à proximité des bords longitudinaux du coussin dans la zone d'entre-jambes et forment des revers longitudinaux orientés verticalement vers l'intérieur et situés plus près des bords longitudinaux du coussin dans la zone d'entre-jambes que de l'axe longitudinal médian et que les parties d'extrémités correspondantes desdits volets forment des zones en recouvrement le long de l'axe longitudinal médian;
- fixer lesdits volets sur leur pourtour à la feuille de support;
- fixer lesdits volets à la feuille de couverture au moyen de lignes de liaison longitudinales situées au-dessus du coussin absorbant dans la zone d'entre-jambes et à l'extérieur desdites boucles; et
- lier entre eux lesdits volets dans les zones en recouvrement des parties d'extrémité desdits volets.

On réalise ainsi un article d'hygiène absorbant, tel qu'une couche-culotte comportant une double barrière d'étanchéité, composée d'une première barrière d'étanchéité périphérique et d'une seconde barrière d'étanchéité latérale, ces deux barrières étant formées au moyen uniquement de deux bandes analogues de matériau.

La suite de la description, qui concerne des réalisations particulières de la présente invention, données à titre d'exemples non limitatifs, se réfère aux figures annexées qui représentent respectivement :
figure 1 une vue de dessus d'une couche-culotte jetable selon la présente invention;
figure 2 une vue en coupe faite suivant la ligne II-II de la figure 1; et
figures 3 et 4 des schémas montrant la variation de forme et de largeur de l'ouverture de la barrière d'étanchéité périphérique en fonction de la hauteur des plis ou revers des volets.

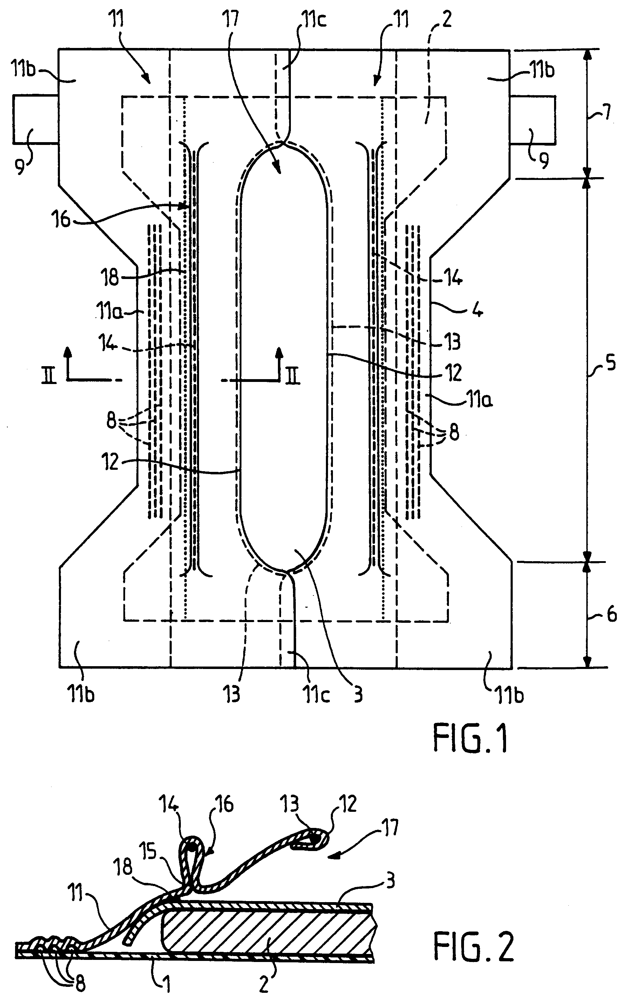

En se référant aux figures 1 et 2, la couche-culotte selon la présente invention comprend de façon classique, une feuille de support extérieure 1 imperméable aux liquides, par exemple formé d'un film mince de polyéthylène, une feuille de couverture intérieure 3 perméable aux liquides, par exemple en non-tissé hydrophile, et un coussin absorbant 2 disposé entre la feuille de support extérieure 1 et la feuille de couverture 3.

De manière classique la feuille de support imperméable 1 présente une forme anatomique avec deux échancrures 4 définissant une zone d'entre-jambes 5 de largeur réduite et des parties d'extrémité avant 6 et arrière 7 de largeur accrue. Comme cela est également classique, le coussin absorbant est de dimension inférieure à la feuille de support extérieure 1 et peut avoir une forme semblable par exemple une forme de sablier, et est fixé à la feuille de support 1, par tous moyens classiques.

La feuille de couverture 3 est généralement de forme rectangulaire et a une largeur réduite par rapport à la feuille de support 1, mais au moins suffisante pour pouvoir recouvrir le coussin absorbant dans la zone d'entre-jambes 5, et elle est fixée au coussin absorbant par des lignes ou zones de collage (non représentées).

Généralement cette feuille de couverture 3 a une longueur au moins égale à la longueur de la zone d'entre-jambes et au plus égale à la longueur totale de la couche-culotte.

La couche-culotte comporte également de façon connue en soi, des premiers éléments élastiques longitudinaux 8 d'entre-jambes et des attaches adhésives 9 sur la partie arrière 7.

La structure décrite ci-dessus est classique et bien connue dans la technique.

Selon l'invention, la couche-culotte comporte en outre deux volets latéraux 11 disposés symétriquement par rapport à l'axe longitudinal médian de la couche-culotte, de forme générale rectangulaire et de même longueur que la couche-culotte. Ces volets 11 sont réunis à la feuille de support le long de leurs bords transversaux et de leur bord longitudinal proximal (ou pourtour) aux bords transversaux et longitudinaux correspondants de la feuille de support 1. Chaque volet comprend une partie intermédiaire 11a de longueur au moins égale à la longueur de la zone d'entre-jambes 5 de la couche-culotte qui relie deux parties d'extrémités 11b. Les parties d'extrémité 11b de chaque volet ont une largeur supérieure à la moitié je la largeur de la couche-culotte de telle sorte que les parties d'extrémité correspondantes forment le long de l'axe longitudinal médian de la couche-culotte deux zones en recouvrement 11c. Les volets sont réunis entre eux dans ces zones en recouvrement par tout moyen approprié, par exemple par collage. La partie intermédiaire 11a de chacun des volets 11 comporte un pli ou revers longitudinal 16 de longueur au moins égale à la longueur de la zone d'entre-jambes 5 et de préférence inférieure à la longueur totale du coussin absorbant 2. Ces plis ou revers 16 sont orientés verticalement vers l'intérieur et sont situés au-dessus du coussin absorbant plus près des bords longitudinaux du coussin dans la zone d'entre-jambes que de l'axe longitudinal médian de la couche-culotte. Des éléments élastiques 14, de préférence de longueur analogue aux plis 16, sont fixés à l'état tendu, aux sommets des plis 16 à l'intérieur de ceux-ci. Les volets 11 sont en outre fixés à la feuille de couverture 3 par une ligne de liaison longitudinale 18 située entre les plis longitudinaux ou revers 16 et le bord longitudinal du coussin 2 dans la zone d'entre-jambes, de préférence le long du bord longitudinal du coussin 2 dans la zone d'entre-jambes 5. Ainsi, les plis longitudinaux 16 se trouvent situés entre les lignes longitudinales de fixation 18 et les bords distaux des volets.

Dans la réalisation recommandée représentée les parties intermédiaires 11a des volets 11 sont de longueur supérieure à la longueur de la zone d'entre-jambes mais inférieure à la longueur du coussin, de sorte que les zones en recouvrement 11c des parties intermédiaires 11b se prolongent depuis les bords transversaux de la couche-culotte jusqu'au-delà des bords transversaux du coussin absorbant.

Dans la réalisation représentée, les plis ou revers 16 de chacun des volets ont une hauteur H pratiquement constante sur toute leur longueur de sorte que les bords longitudinaux distaux 12 des volets forment conjointement sur une majeure partie de leur longueur une ouverture de forme oblongue de largeur pratiquement constante sur une majeure partie de sa longueur. Cette ouverture constitue une première barrière d'étanchéité périphérique formant poche de confinement.

Des éléments élastique 13 sont fixés à l'état tendu, le long d'au moins une majeure partie des bords longitudinaux distaux 12 des volets 11. Dans la réalisation représentée, les bords longitudinaux distaux 12 des volets sont repliés et collés pour former des goussets dans lesquels les éléments élastiques 13, sont fixés à l'état tendu, de préférence à leurs extrémités, par exemple par collage.

Les éléments élastiques peuvent être constitués par un brin unique ou par plusieurs brins parallèles.

Comme indiqué précédemment, la présence des plis ou revers 16 dans les volets 11 éloigne l'un de l'autre les bords longitudinaux distaux 12 des volets 11, excepté au niveau des zones en recouvrement, liées entre elles, des parties d'extrémités créant ainsi une ouverture de forme oblongue 17. Cette ouverture constitue une première barrière d'étanchéité périphérique sur le pourtour du coussin absorbant formant en fait une poche de confinement. D'autre part, les plis ou revers 16 eux-mêmes, qui sont orientés verticalement vers l'intérieur de la couche-culotte et qui sont situés de part et d'autre de l'ouverture 17 constituent une deuxième barrière d'étanchéité latérale située à l'extérieur par rapport à la première barrière d'étanchéité périphérique formant poche de confinement. Avec la construction décrite ci-dessus, on obtient donc une double barrière d'étanchéité comprenant une première barrière d'étanchéité périphérique formant poche de confinement et une deuxième barrière d'étanchéité latérale à l'extérieur de la première barrière d'étanchéité au moyen uniquement de deux volets latéraux et par conséquent avec une consommation minimale de matériau.

Les plis ou revers 16 formés dans les volets 11 peuvent avoir une hauteur constante sur toute leur longueur ou une hauteur qui varie depuis une valeur maximum jusqu'à une valeur minimum aux extrémités des plis. D'autre part, selon la position longitudinale de la hauteur de valeur maximum du pli, la forme et la largeur de l'ouverture 17 varient en correspondance.

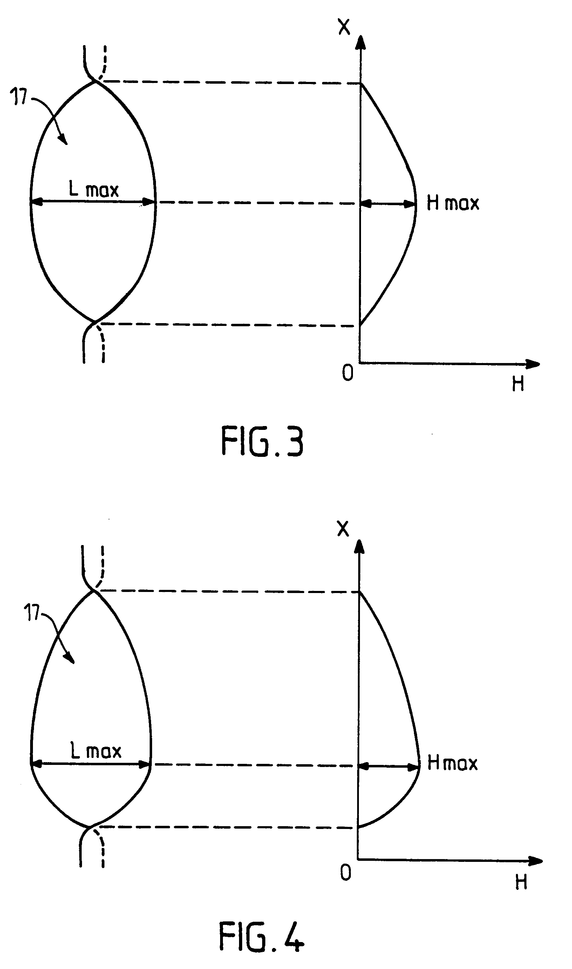

On a représenté aux figures 3 et 4 les variations de forme et de largeur de l'ouverture 17 en fonction de la variation de la hauteur du pli. Sur les figures on a porté en abscisse la valeur de la hauteur du pli et en ordonnée la position longitudinale.

En se référant à la figure 3, on a réalisé un pli 16 dont la hauteur maximum est située au milieu du pli et divise ce pli en deux parties égales dont la hauteur diminue régulièrement depuis le point de hauteur maximale jusqu'aux extrémités du pli. En correspondance, l'ouverture 17 aura la forme symétrique, généralement ovale, représentée dont la largeur maximale est située au milieu en correspondance avec le point de hauteur maximale des plis 16. La largeur de l'ouverture 17 diminue régulièrement de part et d'autre de cette largeur maximale centrale de l'ouverture jusqu'aux parties en recouvrement 11c des volets 11.

Sur la figure 4 on a représenté l'ouverture 17 correspondant à des plis 16 dont le point de hauteur maximale est situé plus près d'une des extrémités du pli. Dans ce cas le point de hauteur maximale des plis divise chacun des plis en deux parties longitudinales inégales, la partie la plus courte ayant une hauteur diminuant plus rapidement jusqu'à l'extrémité correspondante que la partie plus longue. De façon correspondante, l'ouverture 17 présentera une largeur maximale décalée, alignée transversalement avec les points de hauteur maximale des plis 16, formant ainsi une ouverture oblongue, de forme générale ovoïde, dont la largeur diminue régulièrement, mais différemment, vers les zones en recouvrement 11c depuis une valeur maximale, en correspondance avec la diminution de hauteur des plis depuis le point de hauteur maximale.

Ainsi, en faisant varier la hauteur maximale des plis et la position longitudinale de cette hauteur maximale dans les plis, on peut faire varier la forme et la largeur de l'ouverture de la barrière d'étanchéité périphérique de façon à disposer la largeur maximale de l'ouverture à l'endroit appropriée selon l'usage envisagé pour la couche-culotte. Par exemple, on peut prévoir une ouverture plus large vers l'avant pour des couches-culottes destinées à des garçons et une ouverture plus large vers le centre ou vers l'arrière pour des filles.

Ce résultat est obtenu simplement avec la construction de la présente invention, à l'aide uniquement de deux volets latéraux sans qu'il soit nécessaire d'effectuer des découpes ou d'y adjoindre des éléments supplémentaires.

On notera que dans la présente description, les expressions "intérieur" et "extérieur" telles qu'elles sont utilisées pour définir les positions relatives des différents éléments constitutifs de l'article se rapportent à un article porté par un utilisateur, la direction intérieure étant celle orientée vers l'utilisateur et la direction extérieure étant, bien évidemment, celle orientée en éloignement de l'utilisateur.

## Revendications

1. Article d'hygiène absorbant jetable, tel qu'une couche-culotte ou garniture pour incontinent comprenant :
- une feuille de support (1) imperméable aux liquides ayant des bords transversaux et des bords longitudinaux opposés, les bords longitudinaux comportant chacun une échancrure (4) définissant une zone d'entre-jambes (5) de largeur réduite et deux zones d'extrémité (6,7) de largeur accrue;
- un coussin absorbant (2) fixé sur la feuille de support (1) et de dimension inférieure à ladite feuille de support;
- une feuille de couverture (3), perméable aux liquides, de forme générale rectangulaire, fixée au coussin absorbant (2) et de dimension suffisante pour recouvrir ledit coussin absorbant au moins dans la zone d'entrejambe (5);
- des premiers éléments élastiques longitudinaux (8) fixés, à l'état tendu, à la feuille de support (1) à l'extérieur des bords longitudinaux du coussin (2); et
- deux volets latéraux (11), en matériau souple, de forme générale rectangulaire, de longueur supérieure à la longueur du coussin, fixés à la feuille de support (1) symétriquement par rapport à un axe longitudinal médian de l'article;
caractérisé en ce que:
chacun des volets (11) comprend une partie intermédiaire (11a) de longueur au moins égale à la zone d'entrejambes (5) et inférieure à la longueur du coussin absorbant (2), réunissant deux parties d'extrémité (11b);
les parties d'extrémité (11b) d'un volet, sont réunies aux parties d'extrémité (11b) correspondantes de l'autre volet le long de l'axe longitudinal médian;
les parties intermédiaires (11a) de chacun des volets (11) comportent un pli longitudinal (16) identique, de longueur au moins égale à celle de la zone d'entre-jambes (5) et de hauteur (H) déterminée, orienté verticalement vers l'intérieur de l'article et situé au-dessus du coussin absorbant (2) plus près du bord longitudinal correspondant du coussin absorbant que de l'axe longitudinal médian;
des seconds éléments élastiques de longueur au moins égale à celle de la zone d'entre-jambes (5) sont fixés, à l'état tendu, à l'intérieur de chacun des plis longitudinaux (16);
chacun desdits volets (11) est fixé à la feuille de couverture (3) par une ligne de liaison longitudinale (18) située à l'extérieur par rapport audit pli longitudinal (16) et au-dessus du coussin dans la zone d'entre-jambes (5);
de sorte que les bords longitudinaux distaux desdites parties intermédiaires (11a) des volets forment conjointement sur une majeure partie de leur longueur une ouverture longitudinale (17) de forme oblongue au-dessus du coussin absorbant (2), cette ouverture (17) constituant une première barrière d'étanchéité périphérique et les plis (16) constituant une deuxième barrière d'étanchéité latérale située à l'extérieur par rapport à la première barrière.

2. Article selon la revendication 1 caractérisé en ce que les parties d'extrémité (11b) des volets ont une largeur supérieure à la moitié de la largeur de la feuille de support (1) de sorte que les parties d'extrémité correspondantes desdits volets présentent des zones en recouvrement (11c) le long de l'axe longitudinal médian, lesdites zones en recouvrement étant liées entre elles.

3. Article selon la revendication 1 ou 2 caractérisé en ce que chaque volet (11) comprend en outre des troisièmes éléments élastiques longitudinaux fixés, à l'état tendu, le long de l'ouverture (17).

4. Article selon l'une quelconque des revendications 1 à 3 caractérisé en ce que la hauteur (H) déterminée de chacun des plis (16) est constante sur toute la longueur du pli.

5. Article selon l'une quelconque des revendications 1 à 3 caractérisé par le fait que la hauteur (H) déterminée de chacun des plis (16) diminue longitudinalement régulièrement de part et d'autre d'un point de hauteur maximale jusqu'aux extrémités du pli.

6. Article selon la revendication 5 caractérisé en ce que le point de hauteur maximale divise longitudinalement le pli (16) en deux parties égales.

7. Article selon la revendication 5 caractérisé en ce que le point de hauteur maximale divise longitudinalement le pli (16) en deux parties inégales.

8. Article selon l'une quelconque des revendications précédentes caractérisé en ce que lesdits plis (16) sont constitués par une boucle fermée.

9. Procédé de fabrication de volets (11) destinés à être utilisés dans un article d'hygiène absorbant comprenant une feuille de support imperméable aux liquides ayant des bords transversaux et des bords longitudinaux opposés, les bords longitudinaux comportant chacun une échancrure définissant une zone d'entre-jambes de largeur réduite et deux zones d'extrémité de largeur accrue, un coussin absorbant fixé sur la feuille de support et de dimension inférieure à celle-ci, une feuille de couverture, perméable aux liquides, de forme générale rectangulaire, fixée au coussin absorbant et de dimension suffisante pour recouvrir le coussin absorbant au moins dans la zone d'entre-jambes et des premiers éléments élastiques longitudinaux fixés, à l'état tendu, à la feuille de support, à l'extérieur des bords longitudinaux du coussin absorbant, caractérisé en ce qu'il comprend les étapes consistant à :
- réaliser une bande en matériau souple, de forme générale rectangulaire ayant une largeur légèrement supérieure à la moitié de la largeur de l'article d'hygiène, et de longueur analogue à celle de l'article d'hygiène;
- fixer longitudinalement, par rapport à un bord de la bande, à l'état tendu, à une distance inférieure à la moitié de la largeur de la bande, un élément élastique (14) sur une longueur au moins égale à la zone d'entre-jambes de l'article;
- replier la bande sur celle-même autour de l'élément élastique sur une hauteur déterminée (H), pour former une boucle ayant un sommet et des parois latérales longitudinales dont les bords inférieurs longitudinaux sont distants du sommet de ladite hauteur déterminée (H);
- amener en contact lesdits bords inférieurs longitudinaux de la boucle; et
- lier entre eux lesdits bords inférieurs longitudinaux suivant une ligne de liaison longitudinale (15) pour ainsi former un volet (11) ayant une partie intermédiaire (11a), pourvue d'un pli vertical de longueur au moins égale à celle de la zone d'entre-jambes de l'article, réunie à deux parties d'extrémité (11b) de plus grande largeur.

10. Procédé selon la revendication 9 caractérisé en ce que les bords inférieurs des boucles sont liés par collage, thermoscellage ou soudure aux ultrasons.

11. Procédé selon la revendication 9 ou 10 caractérisé par le fait que la bande est constituée d'un non-tissé hydrophobe.

12. Procédé selon l'une quelconque des revendications 9 à 11, caractérisé en ce que la hauteur déterminée (H) dont est repliée la bande est constante sur toute la longueur.

13. Procédé selon l'une quelconque des revendications 9 à 11 caractérisé en ce que la hauteur déterminée (H) dont est repliée la bande diminue régulièrement depuis un point de hauteur maximale.

14. Procédé selon la revendication 13 caractérisé en ce que le point de hauteur maximale divise la boucle longitudinalement en deux parties égales.

15. Procédé selon la revendication 13 caractérisé en ce que le point de hauteur maximale divise la boucle longitudinalement en deux parties inégales.

16. Procédé de fixation de volets latéraux sur un article d'hygiène absorbant comprenant
- une feuille de support (1) imperméable aux liquides ayant des bords transversaux et des bords longitudinaux opposés, les bords longitudinaux comportant chacun une échancrure (4) définissant une zone d'entre-jambes (5) de largeur réduite et deux zones d'extrémité (6,7) de largeur accrue;
- un coussin absorbant (2) fixé sur la feuille de support (1) et de dimension inférieure à ladite feuille de support;
- une feuille de couverture (3), perméable aux liquides, de forme générale rectangulaire, fixée au coussin absorbant (2) et de dimension suffisante pour recouvrir ledit coussin absorbant au moins dans la zone d'entrejambe (5);
- des premiers éléments élastiques longitudinaux (8) fixés, à l'état tendu, à la feuille de support (1) à l'extérieur des bords longitudinaux du coussin (2); et
caractérisé en ce qu'il consiste à :
- réaliser deux volets (11) identiques selon le procédé de l'une quelconque des revendications 9 à 15;
- disposer les volets ainsi réalisés symétriquement par rapport à un axe longitudinal médian de l'article au-dessus de la feuille de support, du coussin absorbant et de la feuille de couverture de telle sorte que les boucles soient orientées verticalement vers l'intérieur et soient situées plus près des bords longitudinaux du coussin dans la zone d'entre-jambes que de l'axe longitudinal médian et les parties d'extrémité (11b) correspondantes des volets forment des zones en recouvrement (11c) le long de l'axe longitudinal médian;
- fixer lesdits volets (11) sur leur pourtour à la feuille de support (1);
- fixer lesdits volets à la feuille de couverture (3) au moyen de lignes de liaison longitudinales (18) situées au-dessus du coussin absorbant dans la zone d'entre-jambes et à l'extérieur par rapport auxdites boucles (16); et
- lier entre eux lesdits volets dans les zones en recouvrement (11c) des parties d'extrémité (11b) desdits volets.

## Claims

1. Disposable absorbent article of hygiene, such as a nappy or dressing for the incontinent, comprising:
- a liquid-impervious supporting sheet (1) which has opposite lengthwise edges and transverse edges, each of the lengthwise edges comprising an indentation (4) defining a crotch region (5) of reduced width and two end regions (6, 7) of increased width;
- an absorbent pad (2) secured to the supporting sheet (1) and smaller in size than the said supporting sheet;
- a liquid-permeable cover sheet (3) of general rectangular shape, secured to the absorbent pad (2) and of sufficient size to cover the said absorbent pad at least in the crotch region (5);
- first lengthwise elastic members (8) secured, in the stretched state, to the supporting sheet (1) outside the lengthwise edges of the pad (2);
- two side flaps (11) made of flexible material, of general rectangular shape and of length greater than the length of the pad and which are secured to the supporting sheet (1) symmetrically in relation to a median lengthwise axis of the article;
characterized in that:
each of the flaps (11) comprises an intermediate part (11a) of a length which is at least equal to the crotch region (5) and smaller than the length of the absorbent pad (2), joining together two end parts (11b);
the end parts (11b) of a flap are joined to the corresponding end parts (11b) of the other flap along the median lengthwise axis;
the intermediate parts (11a) of each of the flaps (11) comprise an identical lengthwise fold (16) of a length which is at least equal to that of the crotch region (5) and of determined height (H), pointing vertically towards the interior of the article and situated above the absorbent pad (2) nearer to the corresponding lengthwise edge of the absorbent pad than to the median lengthwise axis;
second elastic members of a length which is at least equal to that of the crotch region (5) are secured, in the stretched state, to the inside of each of the lengthwise folds (16);
each of the said flaps (11) is secured to the cover sheet (3) by a lengthwise connecting line (18) situated outside in relation to the said lengthwise fold (16) and above the pad in the crotch region (5);
so that the distal lengthwise edges of the said intermediate parts (11a) of the flaps form together over a major part of their length a lengthwise opening (17) of oblong shape above the absorbent pad (2), this opening (17) constituting a first peripheral leakproofing barrier and the folds (16) constituting a second side leak-proofing barrier situated outside in relation to the first barrier.

2. Article according to claim 1, characterized in that the end parts (11b) of the flaps have a width which is greater than half of the width of the supporting sheet (1) so that the corresponding end parts of the said flaps have overlapping regions (11c) along the median lengthwise axis, the said overlapping regions being joined together.

3. Article according to claim 1 or 2, characterized in that each flap (11) additionally comprises third lengthwise elastic members secured, in the stretched state, along the opening (17).

4. Article according to any one of claims 1 to 3, characterized in that the determined height (H) of each of the folds (16) is uniform over the whole length of the fold.

5. Article according to any one of claims 1 to 3, characterized in that the determined height (H) of each of the folds (16) decreases uniformly lengthwise on either side of a point of maximum height as far as the ends of the fold.

6. Article according to claim 5, characterized in that the point of maximum height divides the fold (16) lengthwise into two equal parts.

7. Article according to claim 5, characterized in that the point of maximum height divides the fold (16) lengthwise into two unequal parts.

8. Article according to any one of the preceding claims, characterized in that the said folds (16) consist of a closed loop.

9. Process for the manufacture of flaps (11) intended to be employed in an absorbent article of hygiene comprising a liquid-impervious supporting sheet which has opposite lengthwise edges and transverse edges, each of the lengthwise edges comprising an indentation defining a crotch region of reduced width and two end regions of increased width, an absorbent pad secured to the supporting sheet and smaller in size than the latter, a liquid-permeable cover sheet of general rectangular shape, secured to the absorbent pad and of sufficient size to cover the absorbent pad at least in the crotch region and first lengthwise elastic members secured, in the stretched state, to the supporting sheet, outside the lengthwise edges of the absorbent pad, characterized in that it comprises the stages consisting in:
- producing a strip of flexible material of general rectangular shape which has a width slightly greater than half of the width of the article of hygiene and similar in length to that of the article of hygiene;
- securing an elastic member (14) lengthwise, in relation to one edge of the strip, in the stretched state, at a distance smaller than half of the width of the strip, over a length at least equal to the crotch region of the article;
- folding the strip back onto itself around the elastic member over a determined height (H), to form a loop which has a top and lengthwise side walls whose lengthwise lower edges are distant from the top of the said determined height (H);
- bringing the said lengthwise lower edges of the loop into contact; and
- joining together the said lengthwise lower edges along a lengthwise connection line (15) in order thus to form a flap (11) which has an intermediate part (11a) provided with a vertical fold of a length which is at least equal to that of the crotch region of the article, joined to two end parts (11b) of greater width.

10. Process according to claim 9, characterized in that the lower edges of the loops are joined by adhesive bonding, heat-sealing or ultrasonic welding.

11. Process according to claim 9 or 10, characterized in that the strip consists of a hydrophobic nonwoven.

12. Process according to any one of claims 9 to 11, characterized in that the determined height (H) to which the strip is folded back is uniform over the whole length.

13. Process according to any one of claims 9 to 11, characterized in that the determined height (H) to which the strip is folded back decreases uniformly from a point of maximum height.

14. Process according to claim 13, characterized in that the point of maximum height divides the loop lengthwise into two equal parts.

15. Process according to claim 13, characterized in that the point of maximum height divides the loop lengthwise into two unequal parts.

16. Process for securing side flaps to an absorbent article of hygiene comprising
- a liquid-impervious supporting sheet (1) which has opposite lengthwise edges and transverse edges, each of the lengthwise edges comprising an indentation (4) defining a crotch region (5) of reduced width and two end regions (6, 7) of increased width;
- an absorbent pad (2) secured to the supporting sheet (1) and smaller in size than the said supporting sheet;
- a liquid-permeable cover sheet (3) of general rectangular shape, secured to the absorbent pad (2) and of sufficient size to cover the said absorbent pad at least in the crotch region (5);
- first lengthwise elastic members (8) secured, in the stretched state, to the supporting sheet (1) outside the lengthwise edges of the pad (2); and
characterized in that it consists in:
- producing two identical flaps (11) by the process of any one of claims 9 to 15;
- arranging the flaps thus produced symmetrically in relation to a median lengthwise axis of the article above the supporting sheet, the absorbent pad and the cover sheet, so that the loops are pointing vertically towards the interior and are situated nearer to the lengthwise edges of the pad in the crotch region than to the median lengthwise axis and the corresponding end parts (11b) of the flaps form overlapping regions (11c) along the median lengthwise axis;
- securing the said flaps (11) over their periphery to the supporting sheet (1);
- securing the said flaps to the cover sheet (3) by means of lengthwise connecting lines (18) situated above the absorbent pad in the crotch region and outside in relation to the said loops (16); and
- joining the said flaps together in the overlapping regions (11c) of the end parts (11b) of the said flaps.

## Patentansprüche

1. Hygieneartikel zum Absorbieren von Auswurf, wie Windelhose oder Einrichtung für Inkontinente, enthaltend:
eine für Flüssigkeiten undurchlässige Haltefolie (1) mit gegenüberliegenden Querrändern und Längsrändern, derart, daß die Längsränder jeweils eine bogenförmige Aussparung (4) zum Definieren eines Zwischenbeinbereichs (5) mit verringerter Breite und zwei Randzonen (6, 7) mit erhöhter Größe aufweist;
ein an der Haltefolie (1) fixiertes Saugkissen mit gegenüber der Haltefolie verringerten Abmessungen;
eine für Flüssigkeiten durchlässige Abdeckfolie (3) mit allgemein rechteckiger Form, fixiert an dem Saugkissen, und mit einer zum Abdecken des Saugkissens ausreichenden Abmessung zumindest in dem Zwischenbeinbereich (5);
erste elastische Längselemente (8), die im gehaltenen Zustand an der Haltefolie (1) an der Außenseite der Längsränder des Kissens (2) fixiert sind; und
zwei seitliche Verschlußklappen (11) aus biegsamem Material mit allgemein rechteckiger Form und einer gegenüber der Länge des Kissens höheren Länge, fixiert an dem Haltekissen (1) symmetrisch im Hinblick auf eine Mittenlängsachse des Artikels,
dadurch gekennzeichnet, daß
jede der Verschlußklappen (11) einen Mittenabschnitt (11a) mit einer Länge aufweist, die mindestens gleich derjenigen des Zwischenbeinbereichs (5) und geringer als die Länge des Saugkissens (2) ist, zum Vereinen der beiden Randabschnitte (11b);
die Randabschnitte (11b) der Verschlußklappe durch die zugeordneten Randabschnitte (11b) der anderen Verschlußklappe über die Länge der Längsmittenachse vereint sind;
die Zwischenabschnitte (11a) jeder der Randklappen (11) eine identische Längsfalte (16) aufweist, mit einer Länge, die zumindest gleich derjengien des Zwischenbeinbereichs (5) ist, sowie mit festgelegter Höhe (H), vertikal zu der Innenseite des Artikels ausgerichtet und oberhalb des Saugkissens (2) angeordnet, und zwar näher an dem zugeordneten Längsrand des Saugkissens als an der Mittenlängsachse;
die zweiten elastischen Elemente mit einer Länge mindestens gleich derjenigen des Zwischenbeinbereichs (5) im gespannten Zustand an der Innenseite jeder der Längsfalte (16) fixiert sind;
jede der Verschlußklappen (11) an der Abdeckfolie (3) gemäß einer Verbindungslängslinie (18) fixiert ist, die an der Außenseite bezogen auf die Längsfalte (16) und oberhalb des Kissens im Zwischenbeinbereich (5) angeordnet ist; derart, daß
die beabstandeten Längsränder der Zwischenabschnitte (11a) der Verschlußklappen im zusammengefügten Zustand über ein Hauptteil ihrer Länge eine Längsöffnung (17) mit oberhalb des Saugkissens (2) länglicher Form derart bilden, daß diese Öffnung (17) eine erste Randabdichtbarriere bildet und die Längsfalten (16) eine zweite Randabdichtbarriere bilden, die außerhalb der ersten Barriere liegt.

2. Artikel nach Anspruch 1, dadurch gekennzeichnet, daß die Randabschnitte (11b) der Verschlußklappen eine Länge aufweisen, die höher ist als die Hälfte der Länge der Haltefolie (1) derart, daß die Randabschnitte gemäß den Verschlußklappen Abdeckbereiche (11c) entlang der Mittenlängsachse so bilden, daß die Abdeckbereiche miteinander verbunden sind.

3. Artikel nach Anspruch 1 oder 2, dadurch gekennzeichnet, daß jede Verschlußklappe (11) zudem dritte elastische Längselemente enthält, die in gespanntem Zustand entlang der Öffnung (17) fixiert sind.

4. Artikel nach einem der Ansprüche 1 bis 3, dadurch gekennzeichnet, daß die vorbestimmte Höhe (H) jeder der Falten (16) über die gesamte Länge der Falte konstant ist.

5. Artikel nach einem der Ansprüche 1 bis 3, dadurch gekennzeichnet, daß die festgelegte Höhe (H) jeder der Falten (16) entlang der Längsrichtung regelmäßig ausgehend von einem Punkt mit maximaler Höhe bis zu den Rändern der Falte zu beiden Seiten hin abnimmt.

6. Artikel nach Anspruch 5, dadurch gekennzeichnet, daß der Punkt maximaler Höhe in Längsrichtung die Falte (16) in zwei gleiche Abschnitte teilt.

7. Artikel nach Anspruch 5, dadurch gekennzeichnet, daß der Punkt maximaler Höhe die Falte (16) in Längsrichtung in zwei ungleiche Teile unterteilt.

8. Artikel nach einem der vorhergehenden Ansprüche, dadurch gekennzeichnet, daß die Falten (16) durch eine geschlossene Schlaufe gebildet sind.

9. Verfahren zum Herstellen von Verschlußklappen (11) für den Einsatz bei einem absorbierenden Hygieneartikel mit einer für Flüssigkeiten undurchlässigen Haltefolie mit entgegengesetzten Querrändern und Längsrändern, derart, daß die Längsränder jeweils eine bogenförmige Aussparung zum Definieren eines Zwischenbeinbereichs mit verringerter Größe und zweier Randbereiche mit erhöhter Größe aufweist, ferner ein an der Haltefolie fixiertes Saugkissen mit im Vergleich hierzu geringerer Abmessung, eine für Flüssigkeiten durchlässige und allgemein rechteckförmige Abdeckfolie, die an dem Saugkissen fixiert ist und eine zum Abdecken des Saugkissens zumindest in dem Zwischenbeinbereich ausreichende Abmessung aufweist, und erste elastische Längselemente, die im gespannten Zustand an der Haltefolie fixiert sind, und zwar an der Außenseite der Längsränder des Saugkissens, dadurch gekennzeichnet, daß es folgende Schritte aufweist:
Ausbilden einer Einfassung aus biegsamem Material mit allgemein rechteckförmiger Form und einer im Vergleich zu der Hälfte der Breite des Hygieneartikels leicht größeren Breite und einer Länge, die zu derjenigen des Hygieneartikels analog ist;
längsseitiges Fixieren eines elastischen Elements (14), und zwar an einem Rand der Einfassung im gespannten Zustand mit einer Distanz, die gegenüber der Hälfte der Breite des Bands geringer ist, über eine Länge, die zumindest gleich derjenigen des Zwischenbeinbereichs des Artikels ist;
Falten der Einfassung auf sich selbst um das elastische Element gemäß einer festgelegten Höhe (H) zum Bilden einer Schlaufe mit einem Scheitel sowie seitlicher Längswände, deren oberen Längsränder beabstandet zum Scheitel mit der festgelegten Höhe (H) sind;
Kontaktieren der unteren Längsränder der Schlaufe; und
Verbinden der unteren Längsränder untereinander entlang einer Längsverbindungslinie (15), um hierdurch eine Verschlußklappe (11) mit einem Zwischenabschnitt (11a) zu bilden, versehen mit einer vertikalen Falte einer Länge mindestens gleich derjenigen des Zwischenbeinbereichs und zusammengeführt an den beiden Randabschnitten (11b) mit größerer Breite.

10. Verfahren nach Anspruch 9, dadurch gekennzeichnet, daß die unteren Ränder der Schlaufen durch Kleben, Heißsiegeln oder Ultraschallschweißen verbunden sind.

11. Verfahren nach Anspruch 9 oder 10, dadurch gekennzeichnet, daß das Band durch ein hydrophobes Fadenvlies gebildet ist.

12. Verfahren nach einem der Ansprüche 9 bis 11, dadurch gekennzeichnet, daß die festgelegte Höhe (H), mit der die Einfassung gefaltet ist, über ihre gesamte Länge hinweg konstant ist.

13. Verfahren nach einem der Ansprüche 9 bis 11, dadurch gekennzeichnet, daß die vorbestimmte Höhe (H), mit der die Einfassung gefaltet ist, gleichmäßig ausgehend von einem Punkt maximaler Höhe abnimmt.

14. Verfahren nach Anspruch 13, dadurch gekennzeichnet, daß der Punkt maximaler Höhe die Schlaufe in Längsrichtung in zwei gleiche Teile unterteilt.

15. Verfahren nach Anspruch 13, dadurch gekennzeichnet, daß der Punkt maximaler Höhe die Schlaufe in Längsrichtung in zwei ungleiche Teile unterteilt.

16. Verfahren zum Fixieren von seitlichen Verschlußklappen an einem absorbierenden Hygieneartikel, enthaltend:
eine für Flüssigkeiten undurchlässige Haltefolie (1) mit entgegengesetzten Querrändern und Längsrändern, derart, daß die Längsränder jeweils eine Aussparung (4) zum Festlegen eines Zwischenbeinbereichs (5) mit verringerter Breite aufweisen, sowie zwei Randzonen (6, 7) mit erhöhter Breite;
ein an der Haltefolie (1) fixiertes Saugkissen (2) mit ein im Vergleich zu der Haltefolie verringerter Abmessung;
eine für Flüssigkeiten durchlässige Abdeckfolie (3) mit allgemein rechteckiger Form und fixiert an dem Saugkissen (2) mit einer Abmessung, die zum Abdecken des Saugkissens zumindest in dem Zwischenbeinbereich (5) ausreicht;
erste elastische Längselemente (8), die im gespannten Zustand an der Haltefolie (1) an der Außenseite der Längsränder des Kissens (2) fixiert sind;
dadurch gekennzeichnet, daß es besteht aus den Schritten:
Realisieren der identischen Verschlußklappen (11) gemäß dem Verfahren nach einem der Ansprüche 9 bis 15;
Anordnen der derart realisierten Verschlußklappen in symmetrischer Weise im Hinblick auf die Mittenlängsachse des Artikels oberhalb der Haltefolie, des Saugkissens und der Abdeckfolie derart, daß die Schlaufen vertikal zu der Innenseite hin orientiert sind und näher an den Längsrand des Kissens in dem Zwischenbeinbereich angeordnet sind als an der Mittenlängsachse und daß die Randabschnitte (11b) entsprechend den Schlaufen entlang der Mittenlängsachse Abdeckbereiche (11c) bilden;
Fixieren der Verschlußklappen (11) entlang ihrem Umfang an der Haltefolie (1);
Fixieren der Schlaufen an der Abdeckfolie (3) zumindest an den Längsverbindungslinien (18), die oberhalb des Saugkissens in dem Zwischenbeinbereich angeordnet sind und außerhalb der Schlaufen (16); und
wechselseitiges Verbinden der Verschlußklappen in den Bereichen zum Wiederabdecken (11c) der Randabschnitte (11b) der Verschlußklappen.
